# EUROPEAN PATENT APPLICATION

(11) **EP 2 241 617 A1**
(43) Date of publication of application: **20.10.2010**
(21) Application number: 09157388.1
(22) Date of filing: 06.04.2009
(51) Int. Cl.: C12N 5/074, C12N 5/0775, A61K 35/14

(54) **Multipotent cells derived from blood and methods and uses related thereto**

(71) Applicant: Rijksuniversiteit Groningen, 9712 CP Groningen (NL); ACADEMISCH ZIEKENHUIS GRONINGEN, 9713 GZ Groningen (NL)
(72) Inventor: Harmsen, Martin Conrad, 9713 GZ Groningen (NL)
(74) Representative: Hatzmann, Martin

(57) **Abstract**

The invention relates to the fields of regenerative medicine and tissue engineering. In particular, it relates to a multipotent cell type that is of endothelial progenitor cell origin that can differentiate into various cell types. Provided are isolated multipotent cells derived from blood borne cells that can be induced to differentiate in vivo or ex vivo. Also provided is a method to isolate non-ES multipotent cells, comprising the steps of a) obtaining mononuclear cells from blood an animal; b) establishing a population of adherent cells having an endothelial phenotype; c) allowing the cells obtained in step b) to transdifferentiate into multipotent cells; and d) stabilizing said multipotent cells.

## Description

The invention relates to the fields of regenerative medicine and tissue engineering. In particular, it relates to a multipotent cell type that is of endothelial progenitor cell origin that can transdifferentiate into a multipotent cell and differentiate into various mesodermal and/or ectodermal cell types, such as smooth muscle, pericytes, skeletal muscle, satellite cells, fibroblasts, myofibroblasts, skin, neuronal, glial, tendon, bone and cartilage cell types. It also relates to methods for preparing multipotents cells, a therapeutic agent comprising such cells as active ingredient and a treatment method comprising administering the same.

Tissue- and/or organ damage, as well as the resulting decrease in tissue-and/or organ function, can be obtained as a natural consequence of aging, disease or trauma. Although the body has natural regeneration capacity, in many cases the bodies own regenerative capacity is not sufficient for full restoration of tissue- and/or organ function. For example, after myocardial infarction a inflammatory reaction is mounted that results in the clearance of cell debris and the formation of scar tissue, thereby preserving the maximal myocardial function (Am J Pathol 2007; 170:818-829). However, although the deleterious effects of a myocardial infarction are tempered, the myocardium does not regain its full functional capacity.

Tissue- and/or organ regeneration depends on the recruitment and differentiation of multiple stem cells that differentiate into cells of the tissue in question, as well as the secretion of paracrine mediators that augment these repair, restoration and regeneration processes (Developmental Biology 2000; 221:273-284). However, during disease, the natural processes of tissue regeneration may be hampered by the disease. Hence, a therapeutic need for the induction of tissue regeneration is obvious and can be found in cell therapy (Science 2000; 287:1442-6).

Many adult tissues contain populations of stem and progenitor cells that can self-renew / self-replicate and give raise to daughter cells that mature and will undergo terminal differentiation (Science 2000; 287:1442-6). The best characterized is the hematopoietic stem cell and its progeny, but stem cells are identified in most tissues, including bone marrow (Stem Cells 2001; 19:180-92), mesenchymal tissues (Science 1999; 284:143-7), neuronal tissues (Science 2000; 287:1433-8) and blood vessels (Am J Pathol 2007; 170:1879-92). Mesenchymal stem cells (MSC) are identified as those cells that reside in the bone marrow, which are plastic-adherent and upon culture form fibroblast-like cells that have a differentiation potential into various mesenchymal cell types, including smooth muscle, skeletal muscle, bone and cartilage (Science 1999; 284: 143-7). Mesenchymal progenitor cells, having morphological and phenotypical features and differentiation potential similar to MSC have been described in umbilical cord blood (Br. J. Haemotol 2000; 109:235-42), as well as in adult blood (Stem Cells 2000; 18:252-60), however, at extremely low frequencies.

Repair and regeneration of tissue damage or restoration of tissue function after damage relies on the administration of appropriate numbers of therapeutic cells. Various postnatal tissue-specific and embryonic stem cells (ES) are currently being analyzed as candidate sources for therapeutic intervention for tissue regeneration (Methods Mol Biol 2009; 482:55-90). It has been reported that bone marrow-derived MSC engraft in many organs and differentiate along tissue-specific lineages upon cell transplantation in animal models of disease (Nature Med 2000; 6:1282-6). However, obtaining the quantity of cells needed for human treatment is difficult and utilizes maximal-invasive treatments. ES cells are multipotent cells derived from germinal cells that can be propagated indefinitely in vitro being undifferentiated and induced to form most cell types in vivo (Trends Biotechnol 2000; 18: 53-7). Although ES have been isolated from human, their use in research as well as therapeutic is hampered by ethical considerations (Science 2000; 287:1397).

A prime source of therapeutic cells of non-ES origin is peripheral blood and the endothelial progenitor cell (EPC) is an important cell that can be cultured from peripheral blood. Terminally differentiated mesenchymal-like cells and blood-derived EPC together synergize and amplify therapeutic output (Circ Res 2008; 103: 751-760). To date, the isolation of blood-derived mesenchymal-like therapeutic cells has proven highly variable and inefficient.

The objective of the present invention is therefore to provide a multipotent cell that can differentiate into various cell types, in particular mesodermal and ectodermal cell types such as smooth muscle, pericytes, skeletal muscle, satellite cells, fibroblasts, myofibroblasts, skin, neuronal, glial, tendon, bone and cartilage cell types. Furthermore, it is an objective to be able to provide such cells in sufficient amounts that can be supplied stably with minimum invasion, and without problems such as securing donors and rejection in cell transplantation, and ethical limitations.

The invention provides a solution through the provision of a process for the efficient differentiation of blood-derived cells to multipotent therapeutic cells. The inventors are the first to describe a method for the differentiation of multipotent therapeutic cells from a blood born endothelial progenitor cell. The invention gives different meaning to the concept of multipotency in which several different types of cells differentiate from one particular type of stem cell. The process of the invention involves transdifferentiation which is 'a step back' in developmental stage. More precise, transdifferentiation in biology takes place when a non-stem cell transforms into a different type of cell, or when an already differentiated stem cell creates cells outside its already established differentiation path. In the present invention, a committed stem cell, the EPC, which is destined to differentiate into different types of endothelial cells, reverses to e.g. a multipotent phenotype, which by itself, can differentiate into various different lineages. The invention herewith provides a multipotent cell that can differentiate into various mesodermal and/or ectodermal cell types wherein a sufficient amount can be supplied stably and reproducibly with minimal invasion, void of problems such as rejection in cell transplantation and with low ethical concerns.

Accordingly, the invention provides isolated multipotent cells derived from a blood borne cell, wherein the cells can be induced to differentiate *in vivo* or *ex vivo*. In particular, the multipotent cells can differentiate into mesodermal and/or ectodermal lineages . In a specific aspect, the cells can differentiate at least into mesenchymal cells. Exemplary multipotent cells according to the invention have the capacity to be induced to differentiate to smooth muscle, pericyte, satellite cells, skeletal muscle, myofibroblasts, fibroblasts, skin, neuronal, glial, cartilage, tendon and/or bone cell types.

Multipotent cells according to the invention can be distinguished from cells known in the art by the analysis of specific marker proteins. More specifically, they display no expression of endothelial lineage markers, such as CD144, eNOS, vWF, Tie2 and/or thrombomodulin, no expression of leukocytic cell markers, such as CD11b, CD14, CD45 and/or CD163, and a high expression of mesenchymal lineage markers, such as αSMA, SM22α, SMMHC2 and/or calponin. In one embodiment, the multipotent cells display the phenotype CD31⁻, CD34^{+/-}, CD144⁻, VEGFR-2⁺, eNOS⁻, vWF⁻, Tie2⁻, αSMA⁺, SM22α⁺, SM-MHC2⁺, Calponin⁺, ALK1⁺, ALK5⁺, or any combination thereof. In a preferred embodiment, the cells have the phenotype CD31⁻, CD34^{+/-}, CD144⁻, VEGFR-2⁺, eNOS⁻, vWF⁻, Tie2⁻, αSMA⁺, SM22α⁺, SM-MHC2⁺, Calponin⁺, ALK1⁺, ALK5⁺.

Multipotent cells according to the invention can furthermore be characterized on the basis of their protein secretion profile. In one embodiment, the cells secrete Collagen type 1, Collagen type 3, Fibronectin, Vimentin, Angiopoietin-1, VEGFa, basic FGF, HGF, SDF-1, or any combination thereof.

Circulating smooth muscle progenitor cells (Circulation 2002; 106:1199-204) and circulating monocytes (J Leuk Biol 2003; 74:833-45) which act as multipotent mesenchymal cells have been described in the art (PCT/JP2004/003680). However, these cells do not bare the unique phenotype and protein secretion profile as described in the present invention.

Also provided is a therapeutic agent or composition comprising multipotent cells, optionally in combination with a suitable carrier, like a culture medium and/or scaffold material.

A further aspect relates to a method to isolate non-ES multipotent cells. The method is characterized by the following steps:
a) obtaining mononuclear cells from blood of an animal, preferably a human;
b) establishing a population of adherent cells having an endothelial (progenitor) phenotype
c) allowing the cells obtained in step b) to transdifferentiate into multipotent cells; and d) stabilizing said multipotent cells.

For example, the mononuclear cells are obtained from umbilical cord blood or peripheral blood, preferably peripheral blood. Typically, the animal is a human being. However, the invention is also of use in the field of veterinary research and/or medicine. Major advantages of the starting material being blood borne is that a sufficient amount can be supplied stably and reproducibly with minimal invasion, void of problems such as rejection in cell transplantation and with low ethical concerns.

In step b) a population of adherent cells having an endothelial (progenitor) phenotype is established. This can be achieved by culturing the mononuclear cells on a suitable matrix, for instance a fibronectin-coated surface in a suitable endothelial outgrowth medium. As is exemplified herein below, very good results were achieved using a medium comprising a specific combination of growth factors. Accordingly, in one embodiment step b) comprises culturing mononuclear cells in a culture medium supplemented with the growth factors vascular endothelial growth factor (VEGF), basic fibroblast growth factor (bFGF), insulin-like growth factor (IGF) and hepatocyte growth factor (HGF), each at an effective concentration. This specific combination of growth factors has heretofore never been described for establishing cells having an endothelial phenotype from blood borne cells. It was also found that step b) is negatively influenced by the presence of TGFβ, EGF or a combination thereof. Accordingly, in one embodiment it comprises culturing cells in the presence of VEGF, bFGF, IGF and HGF, and in the absence of TGFβ and EGF.

The person skilled in the art will be able to determine the suitable concentrations at which the growth factors can be used. Typically, it will be at least 1 ng/ml. In a specific embodiment, the concentration of VEGF, preferably VEGF-A, bFGF, HGF and/or IGF, preferably IGF-1, is at least 2 ng/ml, preferably at least 5 ng/ml, for instance 10 ng/ml. HGF may be used at somewhat higher concentrations, for instance at least 10 ng/ml like 15 or 20 ng/ml. It can be critical to use species-matched growth factors, i.e. in case the mononuclear cells are derived from human blood, the growth factors are preferably also of human origin. In addition, in view of the therapeutic *in vivo* application of the cells obtained by the method, it is preferred that all components being contacted with the cells during the course of the method are FDA and/or EMEA approved.

Standard culture media known in the art may be used in the present invention, for example those marketed under the name Medium-199, DMEM or RPMI 1640. Medium-199 was found to be very useful. As will be understood by a person skilled in the art, the culture medium may contain one or more additional ingredients. Exemplary ingredients include heparin, (clinical grade) fetal calf serum (e.g. up to 20%) and conventional antibiotics.

The invention also provides cells having an endothelial (progenitor) phenotype derived from mononuclear cells obtainable by steps a) and b) as defined above. These cells display the phenotype CD31⁺, CD105⁺, VEGFR-2⁺, CD144⁺, Tie2⁺, vWF⁺, eNOS⁺, Telomerase⁺, or a combination thereof.

In step c) of the method, the endothelial-type cells obtained in step b) are allowed to transdifferentiate into multipotent cells. The term transdifferentiate is meant to indicate that the committed endothelial cell obtained in step b), which is destined to differentiate into different types of endothelial cells, reverses to a (mesenchymal) phenotype which by itself can differentiate into various different lineages. It was found that transdifferentiation relied, at least partially, on increased ALK5 kinase activity and/or SMAD2/3 activity, decreased ALK1 kinase activity and/or SMAD1/5/8 activity, or a combination thereof. Transdifferentation is thus suitably achieved by culturing the cells obtained in step b) in a growth medium supplemented with at least one agent capable of inducing at least one agent capable of inducing ALK5 kinase activity and/or SMAD2/3 activity and/or an agent capable of decreasing ALK1 kinase activity and/or SMAD1/5/8 activity,

Suitable agents for use in step c) include Transforming Growth Factor-bèta (TGF-β) (e.g. TGFβ1, β2, β3), Platelet-derived Growth Factor (PDGF), or a combination thereof. Preferably, TGFβ1 is used, more preferably together with PDGF, in particular PDGF-BB. In one embodiment, step c) comprises culturing the cells in the presence of TGFβ1, preferably at a concentration of at least 2 ng/ml and/or PDGF-BB, preferably at a concentration of at least 5 ng/ml. Also here, it was found that certain growth factors have a disturbing effect on the process. Step c) is therefore advantageously performed in the in the absence of bFGF, VEGF, EGF, or a combination thereof. The invention also provides multipotent cells derived from mononuclear cells, obtainable by steps a) through c) as defined in the method described above.

The multipotent cells obtained in step c) display a multipotent yet not very stable phenotype and were not highly viable. The inventors therefore set out to find means and methods to establish a stable multipotent cell culture. It was surprisingly observed that phenotypically stable, self-sustaining multipotent cells could be obtained by culturing the cells from step c) in a culture medium supplemented with bFGF at a concentration of at least 1 ng/ml, like 5 or 10 ng/ml, preferably in combination with TGF-β and/or PDGF. Therefore, also provided are multipotent cells derived from mononuclear cells, obtainable by steps a) through d) as defined herein above.

It will be clear to the skilled person in the art that the methods and cells provided herein, be it the endothelial progenitor-type cell or the (stabilized) multipotent cell can be used for a wide variety of (medical) applications. In one embodiment, cells, or progeny differentiated therefrom, are used in medical or veterinary therapy. Therapy for instance comprises tissue and/or organ regeneration, in particular formation of mesodermal and/or ectodermal tissues, such as cardiovascular tissues, including myocardium, muscle tissue, blood vessels, and/or scar tissue, skin tissue, neural tissue, tendon tissue, bone and/or cartilage, after sustained damage. The cells, or progeny differentiated therefrom, are also very useful for *ex vivo* tissue engineering of replacement tissues such as cardiovascular tissue (including myocardium, blood vessels, muscle flaps, heart valves), skin substitutes, nerve grafts and/or bone replacements. They may also be used as therapeutic agent in wound healing, e.g. in wound contraction devices for the treatment of (congenital) defects. Various therapies for the treatment of chronic wounds have been described. One approach involves using tissue-engineering scaffolds alone, another such approach combines therapeutic cells with a tissue-engineering scaffold.

In another embodiment, the cells, or progeny differentiated therefrom, are used as delivery vehicle for the production and secretion of therapeutic proteins, in particular growth factors and/or extracellular matrix proteins, such as collagens.

Furthermore, the invention provides a method of treating a disease or injury comprising administering to a mammalian subject an amount of the cells according to the invention, or progeny differentiated therefrom, effective to treat the disease or injury. The subject is preferably a human, for instance a human in need of repair and regeneration of tissue damage or restoration of tissue function. The patient-derived i.e. autologous, multipotent cells can be stably and conveniently administered with minimal invasive procedures and will not cause rejection at the time of cell transplantation.

Tissue engineering provides an approach for generating functionally replaceable tissue parts. Cells of the invention can be administered in any suitable manner. They may be administered together with a carrier, like a scaffold. Tissue-engineering scaffolds come in a variety of forms such as weaves, knits, braids, perforated films, meshes, non-wovens, and foams. Scaffolds for tissue-engineering are utilized to provide structure and shape, to guide developing tissues, and to allow cells to attach, proliferate, and differentiate. By definition, tissue-engineering scaffolds are three dimensional, highly porous structures that allow cell and tissue growth and transport of nutrients and waste. Once the newly formed tissue has filled the void, it is desirable to have the scaffold naturally degrade with minimal tissue response. The process of biodegradation can occur by enzymatic cleavage, by surface erosion or by hydrolytic cleavage.

Cells provided herein can be administered by means of a cell-seeded scaffold or a cell-loaded carrier, like hydrogels or microspheres. Other cell types or useful ingredients, such as growth factors, may also be present. Scaffold materials for use in tissue engineering are well known in the art. They comprise scaffolds of synthetic or natural polymers, or blends thereof. Biodegradable scaffolds are used to support and guide the in-growth of cells. The idea is that the scaffolding material eventually disappears leaving only the necessary healthy tissue in a topologically required form. While numerous biodegradable polymers have been investigated as candidates for tissue engineering scaffolds, a vast majority do not meet all the required properties. These characteristics include: proper mechanical properties, tunable degradation rates, cyto-compatibility, and the ability to direct cell growth and differentiation. The typical polymeric candidates for scaffold formation can be separated into two main categories a) natural and b) synthetic. Natural polymers or their derivatives such as chitosan, alginates, fibrin, gelatin, collagen and glycosaminoglycans (GAGs) have been used as scaffolding materials due to their inherent bioregulatory activity. On the other hand, synthetic polymers such as Polylactides, polyglycolides and their copolymer poly-lactic-coglycolic acid (PLGA), polyurethanes, polycaprolactone have also been used, due to their easily tailorable degradation rates and mechanical properties. These two main groups of bio-polymers complement each other in their inherent properties, thus combining natural and synthetic polymers has presented an opportunity to take advantage of each groups desired properties. The shape of the scaffold will vary depending on the intended application. For example, in ex vivo tissue engineering of blood vessels can be performed using a tubular scaffold. Other tissues, e.g. myocard, muscle, may require other shapes.

Also within the scope of the invention are methods for forming tissue engineered constructs without the use of scaffolds. One example of a method may comprise providing a shaped hydrogel negative mold; seeding the mold with cells; allowing the cells to self-assemble in the mold to form a tissue engineered construct

### LEGENDS TO THE FIGURES

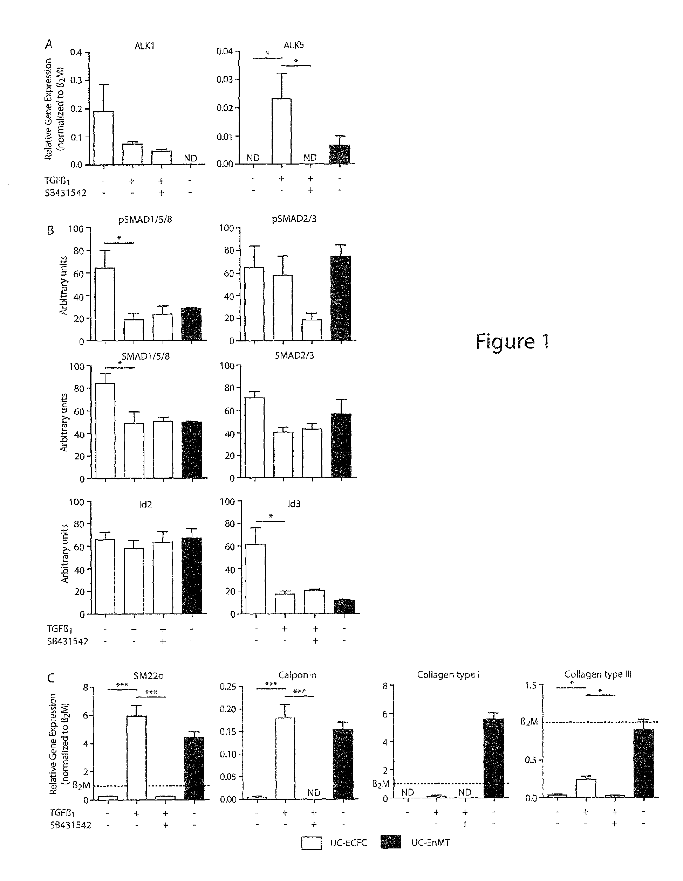

### Figure 1. Transdifferentiation of UC-ECFC is mediated by TGFβ1.

Endothelial-to-mesenchymal transdifferentiation was induced by stimulating UC-ECFC with TGFβ1 for 96h. Activin like kinase 5 (ALK5) kinase inhibitor SB431542 was added to block ALK5 mediated TGFβ signaling.

A, Real-time RT-PCR showed increased gene transcript expression levels of ALK5 after stimulation with TGFβ1. Addition of SB431542 completely inhibited this effect, *P<0.05.

B, Densitometric analysis of SMAD immunoblotting. Basal levels of SMAD1/5/8 were reduced after stimulation with TGFβ1 to levels comparable to those of transdifferentiated cells (EnMT). Phosphorylation of SMAD1/5/8 also decreased after TGFβ1 stimulation. SB431542 did not inhibit these effects. Id2 and Id3 are known to antagonize SMAD2/3 signaling by repressing pSMAD2 mediated gene transcription ²⁸. Id2 levels were stable, in contrast to Id3, which showed strongly reduced levels after stimulation with TGFβ1, *P<0.05.

C, Quantitative RT-PCR showed upregulation of SM22α, calponin and collagen type III. This was completely blocked by SB431542. Expression of collagen type I remained low after 96h of TGFβ1 stimulation compared to levels of transdifferentiated cells, *P<0.05, ***P<0.001.

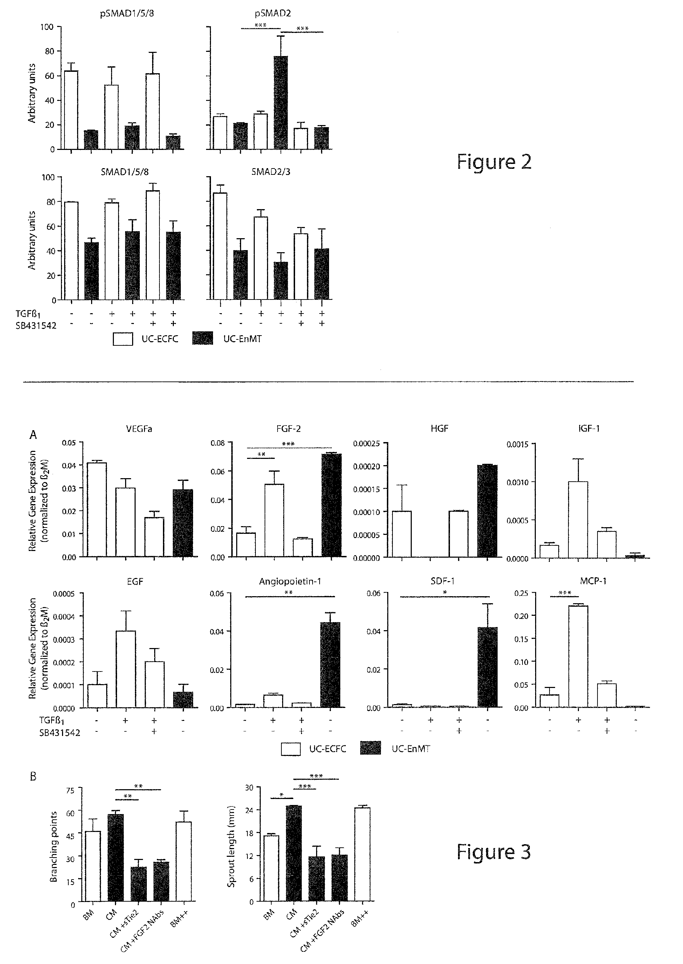

### Figure 2. Transdifferentiated cells (UC-EnMT) show increased responsiveness to TGFβ1.

UC-ECFC and UC-EnMT were stimulated with a pulse of 50ng/mL TGFβ1 for 1h. ALK5 kinase inhibitor SB431542 was added to discriminate ALK5 mediated effects of TGFβ1. Immunoblotting was used to study the expression of basal and phosphorylated SMAD1/5/8 and SMAD2/3. The figure shows the results of densitometric analysis of SMAD immunoblotting. Stimulation of UC-ECFC and UC-EnMT with TGFβ1 had no effect on basal SMAD2/3 and SMAD1/5/8 expression. Levels of pSMAD1/5/8 were reduced in UC-EnMT compared to UC-ECFC, irrespective of TGFβ1 stimulation or ALK5 kinase inhibition. TGFβ1 stimulation induced increased expression of pSMAD2 in transdifferentiated cells, which was completely blocked by SB431542. UC-ECFC did not show increased pSMAD2 levels after stimulation with TGFβ1, ***P<0.001.

### Figure 3. Pro-angiogenic effects of UC-EnMT by paracrine signaling.

A, Quantitative RT-PCR analysis of gene transcript levels of pro-angiogenic factors normalized to β2M expression. Gene transcript levels of basic fibroblast growth factor (FGF-2) and angiopoietin-1 were strongly increased in UC-EnMT compared to UC-ECFC. The increase of FGF-2 expression was induced within 96 hours of TGFβ1 stimulation, *P<0.05, **P<0.01, ***P<0.001.

Capillary sprouting capacity of UC-ECFC was studied using UC-EnMT conditioned medium (CM) with or without FGF-2 neutralizing antibodies (FGF2 NAbs) or recombinant human Tie-2/Fc (sTie2). Unconditioned basal medium (BM) or BM supplemented with 200 ng/mL FGF-2 and 20ng/mL angiopoietin-1 (BM++ )were used as controls. The total numbers of branching points were determined and the cumulative length of spouts was analyzed.

B, The total number of branching points was similar between BM, BM++, and CM. Numbers were reduced with addition of FGF2 NAbs or sTie2 to CM. The cumulative sprout length was increased with CM compared to unconditioned BM, similar to that observed with BM++. Addition of FGF2 NAbs or sTie2 completely diminished these effects, *P<0.05, **P<0.01, ***P<0.001.

### EXPERIMENTAL SECTION

### Summary

During embryogenesis, endothelial cells arise from hemangioblasts ¹. Smooth muscle cells arise from local mesenchyme and the neural crest ^{2;3}. As both vascular cell types originate from different sources, it has long been thought that these cells have distinct progenitors. However, in 2000, Yamashita and colleagues, described embryonic vascular progenitor cells that differentiate into both endothelial and smooth muscle cells ⁴. These results were later confirmed by Ferreira and co-workers, who showed functional integration in preexisting vasculature, of embryonic vascular progenitor cell-derived endothelial cells and smooth muscle cells ⁵. Postnatally, common vascular progenitors have not yet been described. However, reciprocal plasticity between endothelial and mesenchymal lineages, has been shown.

Endothelial-to-mesenchymal transdifferentiation (EnMT) was first described in embryonic development, during formation of the heart valves. ^{6;7}. Later, EnMT of embryonic and adult endothelial cells was also studied *in vitro*, and was shown to be largely TGFβ dependent ⁸⁻¹⁴. The postnatal role of EnMT *in vivo* has long been unclear. Recent evidence supports a role for EnMT in cardiovascular fibrosis ¹⁵. Numerous studies have shown that endothelial progenitor cells (EPC) contribute to neovascularization, either by differentiation into cells of the endothelial lineage and local engraftment into to vasculature ^{16;17} or by secretion of pro-angiogenic factors¹⁹⁻²⁰. In vitro EPC, termed endothelial colony forming cells (ECFC), can be cultured from mononuclear cells from umbilical cord and adult blood ²¹. These cells, with phenotypical and functional characteristics of endothelial cells, show stem cell-like properties, including self-renewal, as observed by the ability of clonal expansion and high telomerase activity ²². The reciprocal plasticity between endothelial and mesenchymal lineages and the stem cell-like properties of ECFC, led us to hypothesize that ECFC, the archetype *in vitro* generated EPC ²¹, can give rise to smooth muscle progeny.

In this example we show the intrinsic capacity of umbilical cord blood-derived endothelial colony forming cells (UC-ECFC) to transdifferentiate into smooth muscle cells; (1) Endothelial-to-mesenchymal transdifferentiation (EnMT) of ECFC resulted in waning of endothelial markers and loss of endothelial functionality. (2) Mesenchymal marker expression and contractile function were gained. (3) EnMT is mediated by ALK5 kinase activity, and characterized by reduced activation of SMAD1/5/8 and reduced levels of Id3, which can be TGFβ1-driven (4) Transdifferentiated cells (UC-EnMT) were phenotypically stable, self-sustaining, independent of exogenously added transdifferentiation mediators, and (5) harbored pro-angiogenic paracrine properties. To our knowledge, this study is the first to describe the plasticity of UC-ECFC towards smooth muscle cell differentiation.

Since their first description ³³, endothelial progenitor cells (EPC) have been extensively studied and their availablity and angiogenic properties have advocated them an interesting and promising cell source for therapeutic neovascularization ³⁴. Despite this large research effort, plasticity of EPC has received little attention.

In vivo, bone marrow-derived smooth muscle progenitor cells (SMPC) have been shown to play a role in cardiovascular pathology ^{35;36}. SMPC have been cultured from umbilical cord and adult blood, although their origin remains largely unclear ^{37;38}. Our results indicate that ECFC, the archetype in vitro EPC ²¹, can also give rise to smooth muscle progeny.

The capacity of ECFC to transdifferentiate to smooth muscle cells certainly has implications for their therapeutic use in cardiovascular disease. The majority of patients eligible for cardiovascular cell therapy commonly share an inflammatory vascular profile ³⁹. TGFβ1 and its downstream effector pSMAD2 have been shown to be highly expressed at atherosclerotic lesion sites ⁴⁰. Recent data from Frutkin *et. al.* indicate that overexpression of TGFβ1 can have antiatherogenic effects. Our data show that increased TGFβ signaling results in mesenchymal differentiation of EPC. This has potential beneficial effects, because transdifferentiated EPC could help limit plaque formation or increase plaque stability ⁴¹. One the other hand, EnMT of EPC could contribute to atherogenesis in an adverse vascular microenvironment ³⁵. Coadministration of smooth muscle progenitor cells and EPC has recently been shown to have synergetic angiogenic effects by increased paracrine signaling, mainly through the angiopoietin/Tie2 signaling pathway ⁴². Our results corroborate these findings and show that angiopoietin-1 mediated pro-angiogenic effects can be exerted by transdifferentiated ECFC as well. In addition, we show that similar pro-angiogenic effects were mediated by FGF-2 secreted by UC-EnMT. ECFC EnMT thereby provides a novel therapeutic strategy for treating ischemic cardiovascular disease. *Ex vivo* priming of ECFC has recently been described to enhance their angiogenic potential ³². Our results illustrate how *ex vivo* stimulation can direct ECFC differentiation, thereby changing phenotypical and functional properties of the cells. This phenomenon can be used in tuning ECFC, creating tailored therapy for use in a specific patient with specific underlying pathology and co-morbidity.

In conclusion, our study demonstrates that ECFC can give rise to smooth muscle progeny by TGFβ1-mediated EnMT. This high plasticity of ECFC clearly has implications for therapeutical use, *e.g*. by providing opportunities for *ex vivo* intervention prior to administration.

### Materials and Methods

### 2.1 Isolation and culture of blood-derived endothelial colony forming cells (UC-ECFC)

Umbilical cord blood was collected at the department of Gynecology, Medical Center Leeuwarden, the Netherlands. Cord blood was isolated directly after normal term delivery, with informed consent from the parents and according to institutional guidelines and the declaration of Helsinki. Cord blood mononuclear cells were separated by density gradient centrifugation on Lymphoprep (Nycomed Pharma, Norway) and cultured on fibronectin-coated (2µg/cm²; Harbor Bio-Products, MA) culture flasks at a density of 1-2·106 cells/cm² in endothelial outgrowth medium containing Medium-199 (Cambrex BioScience, ME), 20% fetal bovine serum (Invitrogen/GIBCO, CA), 1% Penicillin-Streptomycin, 2mM L-glutamine (both Sigma Aldrich, MA), 5U/mL heparin (Leo Pharma, Denmark), 10ng/mL FGF-2, 20ng/mL HGF, 10ng/mL IGF-1 and 10ng/mL VEGFa (all Prepotech, NJ). Cord blood mononuclear cells were cultured for 14 - 21 days before passaging and culture medium was refreshed every third day.

For endothelial-to-mesenchymal transdifferentiation (EnMT), UC-ECFC cultured up to passage 4 were used. EnMT was initiated by replacing the endothelial outgrowth medium with mesenchymal differentiation medium (MDM) consisting of RPMI 1640 supplemented with 20% v/v FCS, 1% Penicillin-Streptomycin, 2mM L-glutamine, 5U/mL heparin, with addition of 5ng/mL TGFβ1 and 15ng/mL PDGF-BB (both from Peprotech, NJ). After 21 days, transdifferentiated cells (UC-EnMT) were cultured in basal medium (BM) consisting of RPMI 1640 supplemented with 20% FCS, 1% Penicillin-Streptomycin, 2mM L-glutamine and 5U/mL heparin, for up to 7 additional passages. When applicable, ALK5-kinase activity was inhibited by addition of 10µM SB-431542 (Sigma, MA) to the culture media.

### 2.2 Phenotypic characterization

Cells were detached using accutase (PAA Laboratories, Austria) and subsequently stained using fluorophore-conjugated antibodies to human CD31 (5µg/mL; IQ Products, The Netherlands), CD34 (5µg/mL; BD Biosciences, CA), CD105 (5µg/mL), CD144 (2.5µg/mL), VEGF-R2 (5µg/mL), Tie-2 (2.5µg/mL), αSMA (2.5µg/mL), TGF6-R2 (5µg/mL), and PDGF-Rβ (5µg/mL; all R&D Systems, MN), or unconjugated antibodies to human eNOS (2.5µg/mL; BD Biosciences, CA), von Willebrand Factor (3µg/mL; DakoCytomation, Denmark), thrombomodulin (10µg/mL), SM22α (5µg/mL), SM-MHC2 (5µg/mL), Calponin (5µg/mL; all Abcam, UK), ALK1 (5µg/mL; R&D Systems, MN) and ALK5 (2µg/mL; Santa Cruz Biotechnology, CA). Unlabeled primary antibodies were subsequently stained using either FITC-conjugated donkey antibody fragments to rabbit IgG (5µg/mL; Jackson ImmunoResearch, PA) or fluoroscein-conjugated rabbit antibodies to mouse IgG (5µg/mL; DakoCytomation, Denmark).

For detection of intracellular proteins, cells were fixed using 2% paraformaldehyde and permeabilized using 0.1% Saponin (Sigma-Aldrich, MA) prior to staining procedures. Flow cytometric analysis was performed on a FACSCalibur™ (BD Biosciences, CA). Fluorophore-conjugated, isotype-matched nonsense antibodies, as well as fluorescein-conjugated donkey antibody fragments to rabbit IgG and fluorescein-conjugated rabbit antibodies to mouse IgG served as controls.

For visualization of cytoskeletal organizations, cells were fixed in ice-cold methanol:acetone (1:1), rehydrated and incubated with 0.1µM fluorescein-conjugated phallotoxins (Molecular Probes/Invitrogen, OR) in phosphate buffered saline (PBS) containing 3µM 4',6-diamidino-2-phenylindole (DAPI; Sigma-Aldrich, MA) for 30 minutes.

For detection of (phosphorylated) SMAD proteins, cells were fixed using 2% paraformaldehyde and permeabilized using 0.1% Saponin (Sigma-Aldrich, MA) prior to staining procedures. Samples were subsequently incubated with antibodies against SMAD2/3 (2µg/mL) and phosphorylated SMAD1/5/8 (pSMAD1/5/8, 2µg/mL; both from Cell Signaling, MA), phosphorylated SMAD2 (pSMAD2, 2µg/mL; Millipore, MA) and SMAD1/5/8 (4µg/mL, Santa Cruz Biotechnology, CA) overnight and incubated the next day using fluorescein-conjugated donkey antibody fragments to rabbit IgG. Labeled samples were visualized using a Leica LMRXA fluorescent microscope and Leica software.

### 2.3 Functional characterizations

To assess the uptake of acetylated low density lipoprotein (acLDL), 10µg/mL DiI-labeled acLDL was added to the culture medium and incubated overnight. Subsequently, cells were fixed using 2% paraformaldehyde (Polysciences, PA), rehydrated and incubated with 3 µM DAPI for 10 minutes.

Binding of lectins from Ulex europaeus (UEA-1) was assessed by incubating 2% paraformaldehyde-fixed cells with fluorescein-conjugated UEA-1 lectins (5µg/mL) dissolved in 3µM DAPI for 1 hour. Subsequently, samples were analyzed on a Leica DMRXA fluorescent microscope at 40x lens magnification.

Capillary sprout formation by UC-ECFC was assessed on MatriGel™ (BD Biosciences, CA). In short, 10µL of MatriGel™ was solidified in µ-Slide Angiogenesis plates (Ibidi GmbH, Germany). Next, 15 000 UC-ECFC were placed on the MatriGel™ and cultured in endothelial outgrowth medium overnight. Sprout formation was analyzed by microscopic analysis.

Thrombin generation was assessed using a Thrombin Generation Assay (HeamoScan, The Netherlands) as described previously ¹. In short, cells were incubated with fibrinogen-depleted plasma under normal culture conditions. A mixture of 30mM CaCl₂ and phospholipids was added to induce the formation of thrombin. At regular intervals, samples were drawn, diluted in ice-cold TrisHCL and incubated with 3mM chromophore-releasing Thrombin substrate S₂₂₃₈. Change in color was assessed in a microtiter plate reader (BioRad, VA) and plotted against a calibration curve of known thrombin concentrations. Human umbilical cord endothelial cells and tissue culture-treated plastic served as negative and positive controls, respectively.

Gel contraction experiments were performed as described previously ². Briefly, a solution of collagen type I (8mg/mL; BD Biosciences, MA) was supplemented with 10mM NaOH, 2.5mM HEPES (GIBCO/Invitrogen, CA) and transdifferentiated cells (1 ·10⁶/mL). Aliquots of 50µL (containing 50 000 cells and 0.2mg collagen type I) were plated on plastic culture dishes and allowed to solidity for 30 minutes. Thereafter 1.5mL culture medium was added, gels were released with a spatula, and imaged using a common flatbed-scanner (ScanJet 5370C; HP, CA). Thereafter, gels were allowed to contract for 48 hours. After 20 hours of spontaneous contraction, TGFβ1 (5ng/mL) was added to the culture medium, and additional contraction was measured after 24h.

### 2.4 Immunoblot analysis

Whole cell lysates (20µg/lane) were electrophoresed in a 10% non-denaturing polyacrylamide gel, blotted onto nitrocellulose, and incubated overnight with primary antibodies. Alkaline phosphatase-conjugated secondary antibodies and NBT/BCIP (Bio-Rad, VA) were used for detection. Densitometric analysis was performed using ImageJ version 1.41 (Research Services Branch, National Institute of Mental Health, Bethesda, MD).

### 2.5 Gene transcript analysis

RNA isolation was performed using the RNeasy Mini Kit (Qiagen Inc., CA) according to manufacturer's protocol. Next, 1µg of total RNA was reverse transcribed using the FirstStrand cDNA synthesis kit (Fermentas UAB, Lithuania) according to manufacturer's instructions. The cDNA-equivalent of 5ng RNA was used for amplification in 384-well microtiter plates in a TaqMan ABI7900HT cycler (Applied Biosystems, CA). Cycle threshold (C_{T}) values for individual reactions were determined and normalized against β2M-expression. Relative expression was calculated using the ΔC_{T}-method. All cDNA samples were amplified in triplicate.

### 2.6 Telomerase expression & activity

Gene expression of Telomerase Reverse Transcriptase (hTERT) was analyzed by RT-PCR. Activity of telomerase was determined using the TeloTAGGG Assay (Roche Diagnostics, the Netherlands) following manufacturers protocol.

### 2.7 Paracrine effects of UC-EnMT

Conditioned medium (CM) was obtained by incubating UC-EnMT in basal medium for 72h. Capillary sprout formation was performed as described in section 2.3 (online data supplement). CM was used with or without 10 µg/mL FGF-2 neutralizing antibodies or 10 µg/mL of recombinant human Tie-2/Fc (both from R&D systems, UK). Unconditioned basal medium or basal medium supplemented with 20 ng/mL FGF-2 and 200 ng/mL angiopoietin-1 (both from Peprotech, NJ), were used as controls. Number of branching points were manually scored and the cumulative length of sprouts was analyzed using ImageJ version 1.41.

### 2.8 Statistical Analysis

All data are expressed as mean ± standard error of mean (SEM). For multiple comparisons testing, one-way ANOVA followed by Tukey *post hoc* analyses were performed. Probabilities (*P*) of less than 0.05 were considered to be statistically significant.

### Results

### Characterization of endothelial colony forming cells (UC-ECFC)

The first UC-ECFC colonies, i.e. a population of adherent cells having an endothelial phenotype, were observed 9-21 days after plating the initial MNC fraction and the cultures reached confluence within the next 5-8 days. Cells were cultured up to an additional three passages after which EC characteristics were determined by binding of fluorescein-conjugated lectin from Ulex europaeus and uptake of DiI-acetylated low density lipoprotein (DiI-acLDL). The ability of UC-ECFC to form capillary-like sprouts on Matrigel was also confirmed. An important functional parameter of EC is their ability to prevent thrombin formation. Indeed, UC-ECFC showed anti-thrombogenic function comparable to human umbilical vein endothelial cells (HUVEC) in an *in vitro* thrombin generation Assay. UC-ECFC phenotype was further characterized by flow cytometric analysis (see Table 1).

**Table 1. Phenotypic description of cell types**

| Marker Protein | UC-ECFC | UC-EnMT |
|---|---|---|
| CD31 | + | - |
| CD34 | +/- | +/- |
| CD105 | + | + |
| CD144 | + | - |
| VEGFR-2 | + | + |
| eNOS | + | - |
| vWF | + | - |
| Thrombomodulin | + | - |
| Tie2 | + | - |
| CD11b | - | - |
| CD14 | +/- | - |
| CD45 | - | - |
| CD163 | - | - |
| αSMA | - | + |
| SM22α | - | + |
| SM-MHC2 | - | + |
| Calponin | - | + |
| ALK1 | + | + |
| ALK5 | +/- | + |
| TGFβR2 | + | + |
| PDGFRb | +/- | + |
| Telomerase | + | +/- |

Marker proteins of the endothelial cell lineage, namely CD31 (99.68±0.25%), CD144 (94.84±1.31%), VEGFR-2 (97.26±2.06%), eNOS (99.08±0.28%), vWF (99.21±0.75%), thrombomodulin (83.73±9.50%) and Tie-2 (90.77±0.85%) were abundantly expressed by UC-ECFC. In contrast, marker proteins of the mesenchymal cell lineage, namely αSMA (0.82±0.70%), SM22α (2.78±2.00%), SM-MHC2 (3.36±2.13%) and calponin (0.05±0.03%), were virtually not expressed by UC-ECFC. UC-ECFC were further analyzed for the expression CD34 (18.84±1.63%) and for the expression of TGFβ-related growth factor receptors. Abundant expression of ALK1 (99.77±0.06%), CD105 (99.90±0.05%) and TGFβ-R2 (78.12±1.42%) was observed on UC-ECFC. In contrast, expression of ALK5 (2.50±0.05%) and PDGF-Rb (4.86±1.92%) was virtually absent.

### Transdifferentiation of UC-ECFC to smooth muscle cells

UC-ECFC were cultured up to passage four in endothelial outgrowth medium, after which they were cultured in mesenchymal differentiation medium (MDM) containing TGFβ1 and PDGF-BB, for an additional 21 days. Transdifferentiated cells (UC-EnMT) had strong proliferative capacity and cultures acquired the classical 'hill and valley' morphology. UC-EnMT had a cytoskeletal organization typical for smooth muscle cells, as shown by staining with phallotoxins. EnMT also resulted in changes in functional properties, *i*.*e*. gain of contractile phenotype and loss of anti-thrombogenicity. Cells were embedded in collagen type I gels and cultured for 20 hours after which a spontaneous contraction of 30,1±5,9% could be observed by the collagen-embedded cells. In contrast, cell-free controls and the gels loaded with UC-ECFC did not show contraction. Addition of TGFβ1 (5ng/mL) for an additional 24h showed additional contraction of the transdifferentiated cell-embedded collagen gels to a total of 72.0±2.3% of control, likely due to TGFβ1 induced Rho activation ²⁴, whereas this had no effect on control gels.

The expression of endothelial lineage markers CD144 (4.06±1.83%), eNOS (0.10±0.10%), vWF (0.50±0.45%), thrombomodulin (4.79±0.35%) and Tie-2 (3.54±1.96%) had diminished after transdifferentiation (Table 1). About 32% of transdifferentiated cells (UC-EnMT) still showed CD31 expression, although the molecular density had decreased by 50-fold. The expression of mesenchymal lineage markers αSMA (96.77±1.43%), SM22α (98.78±1.01%), SMMHC2 (96.30±2.18%) and calponin (90.27±7.09%) was strongly induced during EnMT. After 21 days of EnMT, TGFβ1 and PDGF-BB, were omitted from the culture media. UC-EnMT were cultured up to an additional 7 passages and maintained their mesenchymal phenotype and proliferative behavior. The expression of endothelial lineage marker CD31 was fully lost after this additional culture period. The expression of ALK1 (87.63±1.20%) was reduced 10% after EnMT, while the expression of ALK5 (89.08±6.08%) had increased 35x compared to UC-ECFC levels, resulting in a shift in ALK1/ALK5 balance. Furthermore, EnMT resulted in increased expression of PDGF-Rb (38.59±6.42%).

### Transdifferentiation of UC-ECFC is mediated by TGFβ1.

EnMT was induced by culturing UC-ECFC in MDM, containing TGFβ1 and PDGF-BB, for 96h. SMAD signaling through type 1 TGFβ receptors, ALK1 and ALK5, was analyzed by immunoblotting of the receptor regulated SMADs, SMAD1/5/8 and SMAD2/3. Relative gene expression levels of ALK1 and ALK5, and of SMAD-target genes were determined.

In contrast to UC-ECFC, UC-EnMT had undetectable ALK1 gene transcript levels. ALK1 is involved in activation of the SMAD1/5/8 route through phosphorylation of SMAD1/5/8. In contrast to signaling via ALK5, another type 1 TGFβ-receptor, which leads activation of the SMAD2/3 route ²⁵. ALK5 expression increased after culture in MDM and completely diminished with addition of its kinase activity inhibitor, SB431542, indicative for a positive feedback system (Figure 1A).

UC-ECFC cultured in MDM for 96h showed reduced levels of SMAD2/3 and SMAD1/5/8 protein compared to unstimulated controls. The addition of the ALK5 kinase inhibitor SB431542 had no effect on basal levels of SMAD2/3 and SMAD1/5/8, but resulted in lower levels of pSMAD2 (Figure 1B). Expression levels of pSMAD1/5/8 decreased after culture in MDM and were comparable with UC-EnMT. Supplementing MDM with SB431542, had no effect on the level of SMAD1/5/8 phosphorylation. Given the downregulation of ALK1 and upregulation of ALK5 gene transcription (Figure 1A), physical competition of ALK5 with ALK1 likely caused down-regulation of pSMAD1/5/8 (Figure 1B).

Inhibitor of DNA binding/Differentiation proteins (Id proteins) are dominant negative regulators of basic helix-loop-helix DNA binding transcriptional regulators which play a role in lineage commitment, cell cycle control and cell differentiation ²⁶. Expression of Id genes depends on pSMAD1/5/8-mediated activation of the Id promoters through binding to Smad responsive elements (SREs). In contrast, pSMAD2/3 inhibits Id gene expression through activation of the transcriptional repressor ATF3, which binds to ATF/CREB site on the Id promotors and represses transcription ²⁷. In non-transdifferentiated cells, pSMAD1/5/8 induces Id2 and Id3, which antagonize SMAD2/3 signaling by repressing pSMAD2 mediated gene transcription ²⁸. Ectopic expression of Id2 and Id3 has been shown to inhibit transdifferentiation of epithelial cells ²⁹.

Analysis of Id2 and Id3 protein expression in UC-ECFC showed stable levels of Id2 after culture in MDM. In contrast, Id3 was strongly downregulated in UC-ECFC after stimulation with TGFβ1. This effect was not inhibited by addition of ALK5 kinase inhibitor SB431542 (Figure 1B). Thus, the downregulation of Id3 occurred independent of ALK5 kinase activity or pSMAD2/3.

To study the effect of altered SMAD and Id signaling on induction of EnMT, gene expression levels of several mesenchymal genes were studied. SM22α and calponin increased after 96h of culture in MDM and reached levels similar to UC-EnMT (Figure 1C). SM22α and calponin both are target genes of pSMAD2. However, their increased expression is more likely due to reduced levels of Id3, which, as stated above, acts as a repressor of pSMAD2 mediated gene transcription. Inhibition of ALK5 kinase activity with SB431542 decreased pSMAD2 expression and completely abolished SM22α and calponin gene transcript expression, indicating that both genes are indeed target genes of pSMAD2 (Figure 1C). The gene expression levels of collagen type I was low in cells cultured in MDM for 96 hours compared to expression levels in UC-EnMT. In contrast, gene transcription of collagen type III had increased after 96h of culture in MDM. Transcripts of collagen type I and III genes were low compared to UC-EnMT, and diminished after addition of SB431542. These results indicate a biphasic course of EnMT of ECFC. Within 96h transdifferentiation is induced, shown by downregulation of ALK1, and upregulation of ALK5, SM22α and calponin gene transcription levels. During this induction phase, stimulation with exogenous TGFβ1 is required. In the second phase, gene expression levels of extracellular matrix molecules collagen type I and collagen type III are strongly upregulated, cells show stable mesenchymal phenotypes and are selfsustaining, *i*.*e*. they do not need additional growth factor stimulation.

### UC-EnMT show increased responsiveness to TGFβ1

UC-ECFC and UC-EnMT were stimulated with 50ng/mL of TGFβ1 for 1 hour with or without addition of SB431542. Immunoblotting and immunofluorescent imaging was used to study the expression of inactive, and phosphorylated SMAD2/3 and SMAD1/5/8 (Figures 2A, B and C). Stimulation of UC-ECFC and UC-EnMT with TGFβ1 had no effect on basal SMAD2/3 and SMAD1/5/8 levels (figure 2A and B), while pSMAD1/5/8 levels were clearly reduced in UC-EnMT compared to UC-ECFC. Expression of pSMAD2 increased in TGFβ1-stimulated transdifferentiated cells, and completely diminished with addition of SB431542. UC-ECFC did not show increased pSMAD2 levels after stimulation with TGFβ1 (Figures 2A and B). This differential response to TGFβ1 by UC-ECFC and UC-EnMT is likely due to increased expression of ALK5 in UC-EnMT which leads to increased ALK5 kinase activity and thereby increased phosphorylation of SMAD2 upon TGFβ1 binding. Another explanation might be found in the observed high molecular weight SMAD2/3 complexes present in transdifferentiated cells (figure 2A). Possibly, these preformed complexes allow a faster response to ALK5 stimulation.

### Loss of telomerase activity of UC-ECFC with increasing population doublings and after transdifferentiation.

Gene expression of Telomerase Reverse Transcriptase (hTERT) was analyzed by RT-PCR. UC-ECFC at low population doublings showed a readily detectable gene expression of hTERT, indicating that these cells are capable of self-renewal (Table 1). Subsequently, activity of telomerase was determined in UC-ECFC and UC-EnMT. Telomerase activity of UC-ECFC at low population doubling numbers (<5) was similar to that of hTERT-transfected HUVEC controls. Telomerase activity reduced with increased population doublings, and was undetectable after 20 population doublings. Also after EnMT, telomerase activity was lost.

### Pro-angiogenic effects of UC-EnMT by paracrine signaling.

To study potential paracrine effects of UC-EnMT on UC-ECFC, we analyzed gene transcript levels of several pro-angiogenic factors. Expression levels of HGF, IGF-1 and EGF were low in both UC-ECFC and UC-EnMT. Expression levels of VEGFa were similar in UC-ECFC and UC-EnMT. Interestingly, gene transcript levels of FGF-2 and angiopoietin-1 were strongly increased in UC-EnMT compared to UC-ECFC. In contrast to angiopoietin-1 expression, the increase of FGF-2 expression was induced within 96 hours of TGFβ1 stimulation (Figure 3A).

SDF-1 and MCP-1, are angiogenic factors which play an important role in recruitment and differentiation of EPC ^{20;30-32}. UC-EnMT expressed high levels of SDF-1, which were virtually absent in UC-ECFC, despite TGFβ1 stimulation for 96h. MCP-1 expression, however, was induced by short term TGFβ1 stimulation and almost absent in (quiescent) UC-EnMT. These findings distinguish short-term effects of TGFβ1 stimulation, namely monocyte recruitment by MCP-1, and long-term effects characterized by increased pro-angiogenic cytokine production and SDF-1 mediated recruitment of EPC.

UC-EnMT conditioned medium (CM) was used to study the paracrine effects of UC-EnMT on capillary sprouting of UC-ECFC. Given the increased gene transcript levels of FGF-2 and angiopoietin-1 found in UC-EnMT, neutralizing antibodies to FGF-2 and soluble Tie-2 (sTie-2) were added to CM. The total number of branching points was comparable between basal medium (BM), BM with FGF-2 and angiopoietin-1 (BM++), and CM (46±8, 52±7 and 57±3 respectively). Neutralization of FGF-2 or addition of sTie-2 to the culture medium, resulted in strongly reduced numbers of branching points. The cumulative sprout length was also determined. CM showed increased cumulative length (45.63±1.18%) compared to unconditioned basal medium. BM supplemented with FGF-2 and angiopoietin-1 showed a similar increase in cumulative sprout length (42.66±4.04%). Addition of FGF-2 neutralizing antibodies or sTie-2 completely diminished the observed increase, indicating FGF-2 and angiopoietin-1 mediated effects.

### References

1 Lancrin C,. Nature. 2009;457:892-895.
2 Hellstrom M,. Deuelopment. 1999;126:3047-3055.
3 Hirschi KK, J Cell Biol. 1998;141:805-814.
4 Yamashita J,. Nature. 2000;408:92-96.
5 Ferreira LS, Carc Res. 2007;101:286-294.
6 Markwald RR, Am J Anat. 1977;148:85-119.
7 Mercado-Pimentel ME,. Dev Biol. 2007;304:420-432.
8 DeRuiter MC,. Circ Res. 1997;80:444-451.
9 Arciniegas E, J Cell Sci. 1992;103 ( Pt 2):521-529.
10 Frid MG, Circ Res. 2002;90:1189-1196.
11 Ishisaki A, J Biol Chem. 2003;278:1303-1309.
12 Krenning G, Biomaterials. 2008;29:3703-3711.
13 Paranya G, Am J Pathol. 2001;159:1335-1343.
14 Krenning G,. Trends Cardiovasc Med. 2008;18:314-324.
15 Zeisberg EM,. Nat Med. 2007;13:952-961.
16 Kalka C,. Proc Natl Acad Sci U S A. 2000;97:3422-3427.
17 Yoder MC,. Blood. 2007;109:1801-1809.
18 Krenning G, J Cell Mol Med. 2008.
19 Popa ER, Tissue Eng. 2007;13:2913-2921.
20 Krenning G. Trends Mol Med. 2009;15:180-189.
21 Hirschi KK, Arterioscler Thromb Vasc Biol. 2008;28:1584-1595.
22 Ingram DA, Blood. 2004;104:2752-2760.
23 Moonen JR,. Arthritis Res Ther. 2007;9:R84.
24 Fukata Y, Trends Pharmacol Sci. 2001;22:32-39.
25 Goumans MJ,. Mol Cell. 2003;12:817-828.
26 Norton JD.. J Cell Sci. 2000;113 ( Pt 22):3897-3905.
27 Ruzinova MB, Trends Cell Biol. 2003;13:410-418.
28 Saika S. Am J Physiol Cell Physiol. 2006;290:C282-C289.
29 Kowanetz M,. Mol Cell Biol. 2004;24:4241-4254.
30 Arras M,. J Clin Invest. 1998;101:40-50.
31 De FE,. Blood. 2004;104:3472-3482.
32 Zemani F, Arterioscler Thromb Vasc Biol. 2008;28:644-650.
33 Asahara T, Science. 1997;275:964-967.
34 Rafii S,. Nat Med. 2003;9:702-712.
35 Caplice NM,. Proc Natl Acad Sci U S A. 2003;100:4754-4759.
36 Liu C, Trends Cardiovasc Med. 2004;14:288-293.
37 Le Ricousse-Roussanne Cardiovasc Res. 2004;62:176-184.
38 Simper D,. Circulation. 2002;106:1199-1204.
39 Libby P,. Circulation. 2002;105:1135-1143.
40 Volger OL,. Am J Pathol. 2007;171:326-337.
41 Zoll J, Cardiovasc Res. 2008;77:471-480.
42 Foubert P,. Circ Res. 2008;103:751-760.

## Claims

1. Isolated multipotent cells derived from blood borne cells, wherein the cells can be induced to differentiate *in vivo* or *ex vivo*, preferably wherein the cells can differentiate into mesodermal and/or ectodermal lineages.

2. Multipotent cells according to claim 1, having the capacity to be induced to differentiate to mesodermal and/or ectodermal cell types, such as smooth muscle, pericyte, satellite cells, skeletal muscle, myofibroblasts, fibroblasts, skin, neuronal, glial, cartilage, tendon and/or bone cell types.

3. Multipotent cells according to claim 1 or 2, wherein the cells display the phenotype CD31⁻, CD34^{+/-}, CD144⁻, VEGFR-2⁺, eNOS⁻, vWF⁻, Tie2-, αSMA⁺, SM22α⁺, SM-MHC2⁺, Calponin⁺, ALK1⁺, ALK5⁺, or any combination thereof.

4. Multipotent cells according to any of the preceding claims, wherein the cells secrete Collagen type 1, Collagen type 3, Fibronectin, Vimentin, Angiopoietin-1, VEGFa, basic FGF, HGF, SDF-1, or any combination thereof.

5. A method to isolate non-ES multipotent cells, comprising the steps of:
a) obtaining mononuclear cells from blood of an animal, preferably a human;
b) establishing a population of adherent cells having an endothelial (progenitor) phenotype
c) allowing the cells obtained in step b) to transdifferentiate into multipotent cells; and;
d) stabilizing said multipotent cells.

6. Method according to claim 5, wherein step b) comprises culturing mononuclear cells in a culture medium supplemented with the growth factors VEGF, preferably VEGF-A, bFGF, IGF, preferably IGF-1, and HGF at a concentration of at least 1 ng/ml.

7. Method according to claim 5 or 6, wherein step c) comprises culturing the cells obtained in step b) in a growth medium supplemented with at least one agent capable of inducing ALK5 kinase activity and/or SMAD2/3 activity and/or an agent capable of decreasing ALK1 kinase activity and/or SMAD1/5/8 activity, or a combination thereof.

8. Method according to claim 7, wherein said agent is TGF-β, PDGF, or a combination thereof.

9. Method according to claim 8, comprises culturing the cells in the presence of TGFβ1, preferably at a concentration of at least 2 ng/ml and/or PDGF-BB, preferably at a concentration of at least 5 ng/ml.

10. Method according to any one of claims 5-9, wherein step d) comprises culturing the cells obtained in step c) in a culture medium supplemented with bFGF at a concentration of at least 1 ng/ml.

11. Cells having an endothelial phenotype derived from mononuclear cells, wherein the cells display the phenotype CD31⁺, CD105⁺, VEGFR-2⁺, CD144⁺, Tie2⁺, CD11⁻, CD14^{+/-}, CD45⁻, CD163⁻, vWF⁺, eNOS⁺, Telomerase⁺, or a combination thereof.

12. Multipotent cells derived from mononuclear cells, obtainable by steps a) through c), preferably by steps a) through d), as defined in the method according to any one of claims 5-11.

13. The cells of any one of claims 1-4 or 11-12, or progeny differentiated therefrom for use in medical therapy or veterinary therapy.

14. The cells of claim 13, wherein the medical therapy comprises tissue and/or organ regeneration, in particular formation of mesodermal and/or ectodermal tissues, such as cardiovascular tissues, including myocardium, blood vessels, heart valves, and/or muscle tissue, scar tissue, skin tissue, neural tissue, skin tissue, bone and/or cartilage.

15. Use of the cells of any one of claims 1-4 or 11-12, or progeny differentiated therefrom, for *ex vivo* tissue engineering (such as cardiovascular tissue, including myocardium, blood vessels, muscle flaps, heart valves) of replacement tissues such as skin substitutes, nerve graft and/or bone replacements.

16. Use of the cells of any one of claims 1-4 or 11-12, or progeny differentiated therefrom, as therapeutic agent in wound contraction devices for the treatment of (congenital) defects or as delivery vehicle for the production and secretion of therapeutic proteins, in particular growth factors and collagens.

17. Tissue-engineering scaffold provided with cells according to any one of claims 1-4 or 11-12, or progeny differentiated therefrom.

18. Scaffold according to claim 17, made of a synthetic polymer, a natural polymer or a blend thereof
